# EUROPEAN PATENT APPLICATION

(11) **EP 4 505 985 A1**
(43) Date of publication of application: **12.02.2025**
(21) Application number: 23784803.1
(22) Date of filing: 06.04.2023
(51) Int. Cl.: A61F 13/496, A61F 13/551

(54) **POST-TREATMENT MATERIAL FOR ABSORBENT ARTICLE, ABSORBENT ARTICLE INCLUDING SAID POST-TREATMENT MATERIAL FOR ABSORBENT ARTICLE, AND MANUFACTURING METHOD THEREFOR**

(30) Priority: 08.04.2022 JP 2022064768
(71) Applicant: Nitto Denko Corporation, Ibaraki-shi, Osaka 567-8680 (JP)
(72) Inventor: UCHIDA Sho, Ibaraki-shi, Osaka 567-8680 (JP); IKISHIMA Shinsuke, Ibaraki-shi, Osaka 567-8680 (JP)
(74) Representative: Müller-Boré & Partner Patentanwälte PartG mbB
(86) International application number: PCT/JP2023/014250
(87) International publication number: WO 2023/195519

(57) **Abstract**

Provided is a post-treatment material for an absorbent article, which is excellent in appearance and texture, and can fix and hold a rolled-up state at the time of disposal. The post-treatment material according to an embodiment of the present invention includes: a non-woven fabric layer including a non-woven fabric having a density of from 15 kg/m³ to 120 kg/m³ and containing chemical fibers; a resin layer including an elastomer layer; and a bonding portion to be bonded to an exterior body of an absorbent article. The non-woven fabric layer serves as an outermost surface of the post-treatment material for an absorbent article, and the bonding portion is a portion configured to be weldable by ultrasonic welding or thermal welding.

## Description

### Technical Field

The present invention relates to a post-treatment material for an absorbent article, an absorbent article including the post-treatment material for an absorbent article, and a method of manufacturing the article.

### Background Art

In general, an absorbent article, such as a diaper and a women's sanitary product, includes an absorbing pad for absorbing and holding excreta or the like to be discharged to the outside of the body of a wearer, and an exterior body for supporting the absorbing pad on an inner surface thereof. The absorbent article after use is disposed of after having been fixed in a rolled-up state so that the absorbing pad is positioned on an inner side. As a method of fixing the absorbent article after use, it has been proposed to arrange a post-treatment tape on the absorbent article. The absorbent article is brought into direct contact with a skin, and hence a material that has more satisfactory touch and is flexible is used for the exterior body. Accordingly, there has been proposed such a post-treatment tape that the tape itself is soft and has satisfactory touch (e.g., Patent Literature 1). However, in some cases, the absorbent article after use cannot be sufficiently fixed with the absorbent article using a more flexible material as the exterior body. In addition, the post-treatment tape itself cannot be sufficiently fixed to the exterior body in some cases. Accordingly, there has been required post-treatment means that can fix, in a rolled-up state, even the absorbent article, which has satisfactory touch and in which a flexible material is used for the exterior body, and can dispose of the article.

### Citation List

### Patent Literature

[PTL 1] JP 2021-194256 A

### Summary of Invention

### Technical Problem

The present invention has been made to solve the above-mentioned problems of the related art, and an object of the present invention is to provide a post-treatment material for an absorbent article, which is excellent in appearance and texture, and can fix and hold a rolled-up state at the time of disposal.

### Solution to Problem

According to an embodiment of the present invention, there is provided a post-treatment material for an absorbent article, the post-treatment material including: a non-woven fabric layer including a non-woven fabric having a density of from 15 kg/m³ to 120 kg/m³ and containing chemical fibers; a resin layer including an elastomer layer; and a bonding portion to be bonded to an exterior body of an absorbent article. The non-woven fabric layer serves as an outermost surface of the post-treatment material for an absorbent article, and the bonding portion is a portion configured to be weldable by ultrasonic welding or thermal welding.

In one embodiment, the non-woven fabric is a spunlace non-woven fabric, a carded non-woven fabric, an air-through non-woven fabric, or a spunjet non-woven fabric.

In one embodiment, the chemical fibers are olefin-based resin fibers.

In one embodiment, the chemical fibers are polypropylene-based resin fibers.

In one embodiment, the non-woven fabric contains 30 wt% or more of the polypropylene-based resin fibers.

In one embodiment, the elastomer layer contains a styrene-based elastomer or an olefin-based elastomer.

In one embodiment, the elastomer layer contains the olefin-based elastomer.

In one embodiment, the olefin-based elastomer is a propylene-based elastomer.

In one embodiment, the post-treatment material for an absorbent article has foldable structures.

In one embodiment, the post-treatment material for an absorbent article has a band shape and a length of from 50 mm to 250 mm.

In one embodiment, the post-treatment material for an absorbent article has a basis weight of from 50 g/m² to 150 g/m².

In one embodiment, the post-treatment material for an absorbent article has a breaking elongation of from 150% to 600%.

In one embodiment, the post-treatment material for an absorbent article has a maximum strength of 2 N/10 mm or more.

In one embodiment, the post-treatment material for an absorbent article has a stress after extension of 0.5 N/10 mm or more.

In one embodiment, the post-treatment material for an absorbent article has an elongation at a load of 2 N of 30% or more.

According to another aspect of the embodiment of the present invention, there is provided an absorbent article. The absorbent article includes: an exterior body; and the above-mentioned post-treatment material for an absorbent article.

In one embodiment, in the absorbent article, the exterior body includes a non-woven fabric in an outermost surface thereof, and the non-woven fabric has a density of from 15 kg/m³ to 120 kg/m³.

In one embodiment, the absorbent article is a pants-type diaper.

According to another aspect of the embodiment of the present invention, there is provided a method of manufacturing an absorbent article. The manufacturing method includes: laminating a non-woven fabric layer including a non-woven fabric having a density of from 15 kg/m³ to 120 kg/m³ and containing chemical fibers, and a resin layer including an elastomer layer to produce a laminate; and bonding the laminate and an exterior body of an absorbent article to each other by ultrasonic welding or thermal welding.

### Advantageous Effects of Invention

According to the present invention, the post-treatment material for an absorbent article, which is excellent in appearance and texture, and can fix and hold a rolled-up state at the time of disposal, can be provided.

### Brief Description of Drawings

FIG. **1** is a schematic perspective view of an absorbent article including a post-treatment material for an absorbent article according to an embodiment of the present invention.
FIG. **2** is a schematic sectional view of the post-treatment material for an absorbent article according to the embodiment of the present invention.
FIG. **3** is a schematic sectional view for illustrating a form at the time of use of the post-treatment material for an absorbent article according to the embodiment of the present invention.
FIG. **4** is a schematic perspective view for illustrating the absorbent article fixed with the post-treatment material for an absorbent article according to the embodiment of the present invention.
FIG. **5** is a schematic perspective view for illustrating a rear surface of the absorbent article fixed with the post-treatment material for an absorbent article illustrated in FIG. **4****.**

### Description of Embodiments

### A. Outline of Post-treatment Material for Absorbent Article

A post-treatment material for an absorbent article (hereinafter sometimes referred to as "post-treatment material") according to an embodiment of the present invention includes: a non-woven fabric layer including a non-woven fabric having a density of from 15 kg/m³ to 120 kg/m³ and containing chemical fibers; a resin layer including an elastomer layer; and a bonding portion to be bonded to an exterior body of an absorbent article. The non-woven fabric layer serves as the outermost surface of the post-treatment material for an absorbent article. The bonding portion is a portion configured to be weldable by ultrasonic welding or thermal welding. When the non-woven fabric having a density of from 15 kg/m³ to 120 kg/m³ serves as the outermost surface of the post-treatment material, a post-treatment material excellent in appearance and texture can be provided. In addition, when the bonding portion to be bonded to the exterior body of the absorbent article is a portion configured to be weldable to the exterior body by ultrasonic welding or thermal welding, the post-treatment material can be strongly fixed to the absorbent article. The post-treatment material according to the embodiment of the present invention is easily extended and hence easily subjected to post-treatment at the time of use, and can hold the absorbent article in, for example, a rolled-up state after fixing. The post-treatment material according to the embodiment of the present invention typically has a band shape. The bonding portion is arranged on, for example, each of both the end portions of the post-treatment material having a band shape.

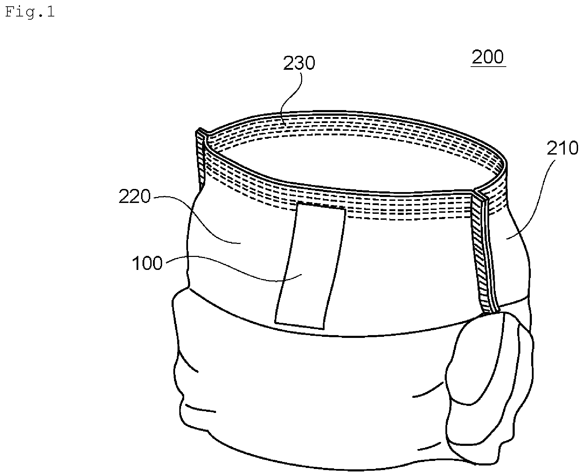

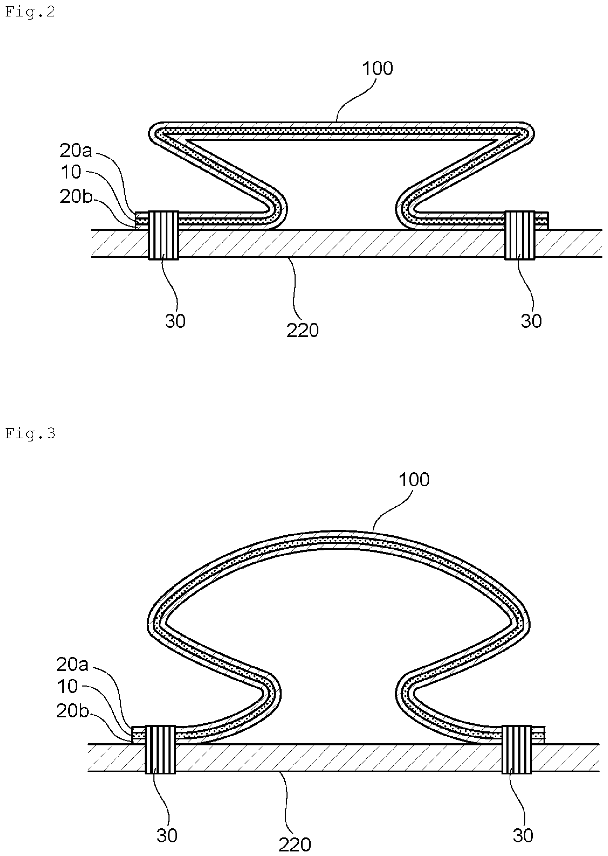

FIG. 1 is a schematic perspective view of the absorbent article including the post-treatment material for an absorbent article according to the embodiment of the present invention. In the illustrated example, an absorbent article **200** is a pants-type diaper. The absorbent article **200** that is the pants-type diaper includes an exterior body including an external member **210** on a ventral side of a wearer and an external member **220** on a dorsal side thereof. FIG. **2** is a schematic sectional view of the post-treatment material for an absorbent article according to the embodiment of the present invention. A state in which bonding portions **30** are bonded to the dorsal external member **220** of the exterior body is illustrated in the illustrated example. In the illustrated example, a space is present between a post-treatment material **100** and the dorsal external member **220** for describing the configuration of the post-treatment material according to the embodiment of the present invention. In an actual usage mode, however, the folded post-treatment material **100** and the exterior body (e.g., the dorsal external member **220**) may be brought into contact with each other until the material is used as the post-treatment material. In the illustrated example, the post-treatment material **100** for an absorbent article has a band shape, and includes: a resin layer **10** including an elastomer layer; and non-woven fabric layers **20a** and **20b.** The non-woven fabric layer **20a** is a layer serving as the outermost surface of the post-treatment material **100,** and includes a non-woven fabric having a density of from 15 kg/m³ to 120 kg/m³ and containing chemical fibers. When such laminated structure is present, a post-treatment material including a bonding portion configured to be weldable to the absorbent article by ultrasonic welding or thermal welding is obtained. In addition, when the non-woven fabric serves as the outermost surface, a post-treatment material excellent in appearance and texture can be provided. In the illustrated example, the non-woven fabric layers are present on both the sides of the resin layer **10** including the elastomer layer, but the non-woven fabric layer **20b** on an exterior body side of the absorbent article may be omitted. The post-treatment material **100** includes the bonding portions **30** to be bonded to the exterior body of the absorbent article (e.g., the dorsal external member **220**). In the illustrated example, the bonding portions **30** are arranged on both the ends of the post-treatment material **100** in its length direction. In the illustrated example, the dorsal external member **220** of the absorbent article **200** is a non-woven fabric. When the exterior body is a non-woven fabric, particularly a non-woven fabric excellent in flexibility, the post-treatment material **100** and the exterior body cannot be strongly fixed to each other in some cases. In the case where the bonding portions **30** are weldable by ultrasonic welding or thermal welding, the post-treatment material and the absorbent article can be strongly fixed to each other even when the exterior body is a non-woven fabric excellent in flexibility. The exterior body supports an absorbing pad for absorbing excreta or the like on its inner side (side to be brought into contact with the wearer) (not shown). In the illustrated example, the post-treatment material **100** is bonded to an upper portion of the dorsal external member **220** (a waist portion **230** side of the absorbent article 200). The post-treatment material **100** is bonded to any appropriate position of the absorbent article **200** in accordance with a size or the like.

In the illustrated example, the post-treatment material **100** has a band shape. The post-treatment material **100** having a band shape has two foldable structures each having a Z-shape (right side in the illustrated example) or a reverse Z-shape (left side in the illustrated example) at both ends. The post-treatment material **100** having foldable structures is excellent in handleability of the absorbent article until use, and when used in post-treatment, can develop the post-treatment material and easily fix the post-treatment material after use.

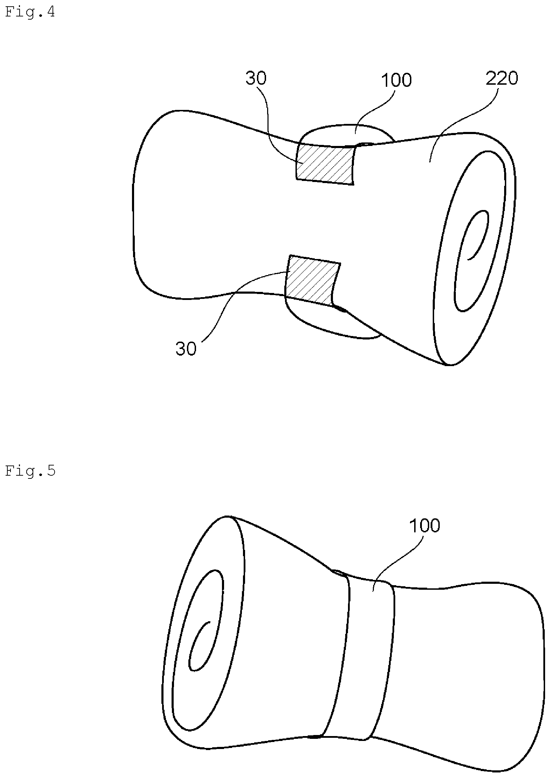

An example of a post-treatment method including using the post-treatment material according to the embodiment of the present invention is described. FIG. **3** is a schematic sectional view for illustrating a form at the time of use of the post-treatment material for an absorbent article according to the embodiment of the present invention. In the illustrated example, at the time of the post-treatment, the foldable structures are developed in the post-treatment material **100** by extending a portion free from being fixed to the dorsal external member **220** to an upper portion (e.g., in a direction opposite to the exterior body). The developed post-treatment material **100** and the dorsal external member **220** of the exterior body form a cyclic structure. As described above, the post-treatment material **100** includes the resin layer **10** including an elastomer layer. The post-treatment material **100** including the resin layer **10** including an elastomer layer can be extended longer than its original length by being further extended after development. As a result, a hollow portion of the cyclic structure formed by the post-treatment material **100** and the dorsal external member **220** can be enlarged, and hence a rolled-up absorbent article can be easily passed through the cyclic structure. FIG. **4** is a schematic perspective view for illustrating the absorbent article fixed with the post-treatment material for an absorbent article according to the embodiment of the present invention. FIG. **5** is a schematic perspective view for illustrating a rear surface of the absorbent article fixed with the post-treatment material for an absorbent article illustrated in FIG. **4****.** When the extension of the post-treatment material **100** is stopped, the length of the extended post-treatment material is returned to its original length by the recovery of elasticity of the resin layer (specifically, the elastomer layer in the resin layer). As a result, the rolled-up absorbent article can be fixed in a rolled-up state, and hence can be easily disposed of. As illustrated in FIG. **4****,** the post-treatment material **100** is bonded to the dorsal external member **220** by the bonding portions **30,** and hence a state in which the absorbent article and the post-treatment material are bonded to each other can be satisfactorily maintained even when the post-treatment material **100** is pulled for fixing. In addition, as illustrated in FIG. **5****,** a state in which the vicinity of the center of the rolled-up absorbent article is fixed with the post-treatment material **100** in which the elasticity has been recovered is maintained on a surface on a side opposite to the bonding portions **30,** and hence, for example, the following case can be suppressed: the fixed state is loosen and the absorbing pad in the inner side is exposed at the surface at the time of disposal.

The post-treatment material **100** is set to any appropriate size that can fix the absorbent article after use, and that can fix and hold the rolled-up state at the time of disposal. As described above, in one embodiment, the post-treatment material **100** has a band shape. The length of the post-treatment material having a band shape is preferably 50 mm or more, more preferably 70 mm or more, still more preferably 100 mm or more. In addition, the length of the post-treatment material having a band shape is preferably 250 mm or less, more preferably 230 mm or less, still more preferably 200 mm or less. In one embodiment, the length of the post-treatment material having a band shape is preferably from 50 mm to 250 mm, more preferably from 70 mm to 230 mm, still more preferably from 100 mm to 200 mm. When the length of the post-treatment material is less than 50 mm, the hollow portion becomes small, and hence there is a risk in that the fixation of the rolled-up absorbent article becomes difficult. When the length of the post-treatment material is more than 250 mm, productivity of the post-treatment material may be reduced and cost therefor may be increased. The width of the post-treatment material is, for example, 8 mm or more, preferably 9 mm or more. The width of the post-treatment material is, for example, 30 mm or less, preferably 25 mm or less. In one embodiment, the width of the post-treatment material is, for example, from 8 mm to 30 mm, preferably from 9 mm to 25 mm. When the width of the post-treatment material falls within the above-mentioned ranges, the rolled-up absorbent article can be fixed and held.

The basis weight of the post-treatment material is preferably 50 g/m² or more, more preferably 70 g/m² or more, still more preferably 90 g/m² or more. In addition, the basis weight of the post-treatment material is preferably 150 g/m² or less, more preferably 130 g/m² or less, still more preferably 120 g/m² or less. In one embodiment, the basis weight of the post-treatment material is from 50 g/m² to 150 g/m², more preferably from 70 g/m² to 130 g/m², still more preferably from 90 g/m² to 120 g/m². When the basis weight of the post-treatment material is less than 50 g/m², there is a risk in that the strength of the post-treatment material is not sufficiently obtained. In addition, the productivity of the post-treatment material may be reduced. When the basis weight of the post-treatment material is more than 150 g/m², cost therefor may be increased.

The breaking elongation of the post-treatment material is preferably 150% or more, more preferably 170% or more, still more preferably 180% or more. In addition, the breaking elongation of the post-treatment material is preferably 600% or less, more preferably 580% or less, still more preferably 560% or less. In one embodiment, the breaking elongation of the post-treatment material is preferably from 150% to 600%, more preferably from 170% to 580%, still more preferably from 180% to 560%. When the breaking elongation falls within the above-mentioned ranges, the post-treatment material can be easily developed and extended, and hence the absorbent article can be easily fixed. Herein, the breaking elongation refers to a breaking elongation when a sample having been cut into a piece having a width of 10 mm and a length of 10 cm so that its direction perpendicular to a machine direction of the post-treatment material serves as a long side is set in a tension testing machine at a chuck-to-chuck distance of 50 mm, followed by pulling at a tensile speed of 500 mm/min until its breakage.

The maximum strength of the post-treatment material is preferably 2 N/10 mm or more, more preferably 3 N/10 mm or more, still more preferably 4 N/10 mm or more. When the maximum strength falls within the above-mentioned ranges, damage to the post-treatment material at the time of the post-treatment can be suppressed. The maximum strength of the post-treatment material is preferably as large as possible, and is, for example, 50 N/10 mm or more. In one embodiment, the maximum strength of the post-treatment material is preferably from 2 N/10 mm to 50 N/10 mm, more preferably from 3 N/10 mm to 40 N/10 mm, still more preferably from 4 N/10 mm to 30 N/10 mm. Herein, the maximum strength refers to a maximum strength when a sample having been cut into a piece having a width of 10 mm and a length of 10 cm so that its direction perpendicular to a machine direction of the post-treatment material serves as a long side is set in a tensile testing machine at a chuck-to-chuck distance of 50 mm, followed by pulling at a tensile speed of 500 mm/min until its breakage.

The stress after extension of the post-treatment material is preferably 0.5 N/10 mm or more, more preferably 0.7 N/10 mm or more, still more preferably 0.8 N/10 mm or more. In addition, the stress after extension of the post-treatment material is preferably 4 N/10 mm or less, more preferably 3 N/10 mm or less. In one embodiment, the stress after extension of the post-treatment material is preferably from 0.5 N/10 mm to 4 N/10 mm, more preferably from 0.7 N/10 mm to 4 N/10 mm, still more preferably from 0.8 N/10 mm to 3 N/10 mm. When the stress after extension falls within the above-mentioned ranges, a state in which the rolled-up absorbent article is fixed can be satisfactorily maintained. Herein, the stress after extension refers to a stress when: the sample having been cut into a piece having a width of 10 mm and a length of 10 cm so that the direction perpendicular to the machine direction of the post-treatment material serves as a long side is set in the tensile testing machine at a chuck-to-chuck distance of 50 mm; the sample is extended at a tensile speed of 500 mm/min until an elongation of 100% is achieved, and is held for 10 minutes; and then the distance is returned to the initial chuck-to-chuck distance.

The elongation at a load of 2 N of the post-treatment material is preferably 30% or more, more preferably 35% or more, still more preferably 40% or more. In addition, the elongation at a load of 2 N is preferably 200% or less, more preferably 150% or less, still more preferably 100% or less. In one embodiment, the elongation at a load of 2 N of the post-treatment material is preferably from 30% to 200%, more preferably from 35% to 150%, still more preferably from 40% to 100%. When the elongation at a load of 2 N falls within the above-mentioned ranges, the post-treatment material can be easily developed, and hence the absorbent article can be easily fixed. Herein, the elongation at a load of 2 N refers to an elongation when: the sample having been cut into a piece having a width of 10 mm and a length of 10 cm so that the direction perpendicular to the machine direction of the post-treatment material serves as a long side is set in the tensile testing machine at a chuck-to-chuck distance of 50 mm; and the sample was extended at a tensile speed of 500 mm/min until a load becomes 2 N.

The thickness of the post-treatment material may be set to any appropriate value. The thickness of the post-treatment material is preferably 0.5 mm or more, more preferably 0.7 mm or more, still more preferably 0.8 mm or more. In addition, the thickness of the post-treatment material is preferably 2 mm or less, more preferably 1.5 mm or less, still more preferably 1.2 mm or less. In one embodiment, the thickness of the post-treatment material is preferably from 0.5 mm to 2 mm, more preferably from 0.7 mm to 1.5 mm, still more preferably from 0.8 mm to 1.2 mm. When the thickness falls within the above-mentioned ranges, the post-treatment material can be suitably used as a member of the absorbent article. Herein, the thickness of the post-treatment material refers to a value obtained by photographing a cross-section of the post-treatment material with a digital microscope (e.g., a digital microscope "VHX-1000" manufactured by Keyence Corporation), followed by measurement with the image analysis software. In the measurement of the thickness, when the non-woven fabric serves as the outermost surface of the post-treatment material, parallel lines drawn so as to have 10 points of intersection with fibers in a cross section on a non-woven fabric side (an upper portion and a lower portion of the post-treatment material) are defined as an upper side and a lower side, respectively, and a length between the upper side and the lower side is adopted as the thickness of the post-treatment material.

### B. Non-woven Fabric Layer

The non-woven fabric layer is a layer serving as the outermost surface of the post-treatment material. The non-woven fabric layer includes a non-woven fabric having a density of from 15 kg/m³ to 120 kg/m³ and containing chemical fibers. It is preferred that the non-woven fabric having a density of from 15 kg/m³ to 120 kg/m³ and containing chemical fibers serve as the outermost surface of the post-treatment material. The non-woven fabric is excellent in flexibility and texture. In addition, the non-woven fabric matches the non-woven fabric to be used for the exterior body of the absorbent article when bonded to the absorbent article, and is excellent in appearance. The non-woven fabric layer may be a single layer, or may be a laminate having two or more layers. When the non-woven fabric layer is a laminate having two or more layers, the non-woven fabric serving as the outermost surface only needs to be a non-woven fabric having a density of from 15 kg/m³ to 120 kg/m³ and containing chemical fibers.

The non-woven fabric is preferably a spunlace non-woven fabric, a carded non-woven fabric, an air-through non-woven fabric, or a spunjet non-woven fabric. When such non-woven fabric is adopted, the surface of the non-woven fabric is excellent in appearance. In addition, the non-woven fabric can match the exterior body of the absorbent article.

As described above, the non-woven fabric is a non-woven fabric containing chemical fibers. When the non-woven fabric of the chemical fibers is adopted, the non-woven fabric can be satisfactorily bonded to the exterior body of the absorbent article by ultrasonic welding or thermal welding. As a raw material for the chemical fibers, there are given, for example: an olefin, such as polypropylene or polyethylene; polyester such as polyethylene terephthalate; polyamide; polyurethane; rayon; nylon; acrylic; a copolymer thereof; and a blend thereof. Fibers of olefin-based resins, such as polypropylene and polyethylene, may be preferably used, and polypropylene-based resin fibers are more preferably used. When a non-woven fabric containing the olefin-based resin fibers is adopted, a chemical odor can be suppressed, and the non-woven fabric can be satisfactorily bonded to the exterior body of the absorbent article.

The non-woven fabric contains preferably 30 wt% or more, more preferably 45 wt% or more, still more preferably 80 wt% or more of the polypropylene-based resin fibers with respect to the fibers for forming the non-woven fabric. The non-woven fabric may contain 100 wt% of the polypropylene-based resin fibers as the fibers for forming the non-woven fabric. In one embodiment, the non-woven fabric contains 30 wt% to 100 wt%, more preferably 45 wt% to 100 wt%, still more preferably 80 wt% to 100 wt% of the polypropylene-based resin fibers with respect to the fibers for forming the non-woven fabric. When the content of the polypropylene-based resin fibers falls within the above-mentioned ranges, a chemical odor can be suppressed, and the non-woven fabric can be satisfactorily bonded to the exterior body of the absorbent article.

The fibers for forming the non-woven fabric may each contain any appropriate other component to the extent that the effects of the present invention are not impaired. Examples of such other component include any other polymer, a tackifier, a plasticizer, an antidegradant, a pigment, a dye, an antioxidant, an antistatic agent, a lubricant, a foaming agent, a heat stabilizer, a light stabilizer, an inorganic filler, and an organic filler. The number of kinds of those components may be only one, or two or more. The content of the other component is preferably 10 wt% or less, more preferably 7 wt% or less, still more preferably 5 wt% or less, particularly preferably 2 wt% or less, most preferably 1 wt% or less.

The density of the non-woven fabric is from 15 kg/m³ to 120 kg/m³. The density of the non-woven fabric is preferably 20 kg/m³ or more, more preferably 23 kg/m³ or more. In addition, the density of the non-woven fabric is preferably 115 kg/m³ or less, more preferably 110 kg/m³ or less. When the density of the non-woven fabric falls within the above-mentioned ranges, a post-treatment material excellent in appearance and texture is obtained. In one embodiment, the density of the non-woven fabric is preferably from 20 kg/m³ to 115 kg/m³, more preferably from 23 kg/m³ to 110 kg/m³. Herein, the density (kg/m³) of the non-woven fabric refers to a value calculated from a basis weight (X g/m²) of the non-woven fabric and a thickness (Y mm) of the non-woven fabric measured based on a method to be described later. More specifically, the density (kg/m³) of the non-woven fabric is calculated as X/Y (kg/m³).

Method of measuring thickness of non-woven fabric: A cross-section of the post-treatment material is photographed with a digital microscope (e.g., a digital microscope "VHX-1000" manufactured by Keyence Corporation), and the thickness of the non-woven fabric is measured with image analysis software. In the measurement of thickness, when the resin layer and the non-woven fabric are brought into contact with each other, the boundary between the resin layer and the non-woven fabric is defined as the outermost surface, or when the non-woven fabrics are brought into contact with each other, in the upper portion and lower portion of the cross-section of the non-woven fabric, parallel lines drawn so as to have 10 points of intersection with fibers are defined as an upper side and a lower side, respectively, and a length between the upper side and the lower side is adopted as the thickness of the non-woven fabric.

The basis weight of the non-woven fabric is preferably 15 gsm (g/m²) or more, more preferably 20 gsm or more, still more preferably 25 gsm or more. In addition, the basis weight of the non-woven fabric is preferably 50 gsm or less, more preferably 45 gsm or less, still more preferably 40 gsm or less. In one embodiment, the basis weight of the non-woven fabric is from 15 gsm to 50 gsm, more preferably from 20 gsm to 45 gsm, still more preferably from 25 gsm to 40 gsm. The basis weight of the non-woven fabric may be measured by any appropriate method. The measurement may be performed by, for example, the following method. The basis weight of the non-woven fabric may be measured by: measuring the weight of a sample obtained by cutting the non-woven fabric into an area of 100 cm² with an electronic balance; and converting the measured value into the unit of g/m² (gsm).

### C. Resin Layer including Elastomer Layer

The resin layer including an elastomer layer may be formed only of an elastomer layer, or may be a laminate of an elastomer layer and any other resin layer. The other resin layer may include any appropriate resin, and includes, for example, a non-elastomeric resin. In one embodiment, the resin layer has a three-layer structure including an elastomer layer and surface layers arranged on both the sides of the elastomer layer.

The elastomer layer is formed by using any appropriate elastomer resin. Examples of the elastomer resin serving as a main component of the elastomer layer include an olefin-based elastomer, a styrene-based elastomer, a vinyl chloride-based elastomer, a urethane-based elastomer, an ester-based elastomer, and an amide-based elastomer. Of those, an olefin-based elastomer or a styrene-based elastomer is preferably used.

When the olefin-based elastomer is used, the post-treatment material and the exterior body can be satisfactorily bonded to each other, and a chemical odor from the post-treatment material can be suppressed. Examples of the olefin-based elastomer include an olefin block copolymer, an olefin random copolymer, an ethylene copolymer, a propylene copolymer, an ethylene olefin block copolymer, a propylene olefin block copolymer, an ethylene olefin random copolymer, a propylene olefin random copolymer, an ethylene propylene random copolymer, an ethylene (1-butene) random copolymer, an ethylene (1-pentene) olefin block copolymer, an ethylene (1-hexene) random copolymer, an ethylene (1-heptene) olefin block copolymer, an ethylene (1-octene) olefin block copolymer, an ethylene (1-nonene) olefin block copolymer, an ethylene (1-decene) olefin block copolymer, a propylene ethylene olefin block copolymer, an ethylene (α-olefin) copolymer, an ethylene (α-olefin) random copolymer, an ethylene (α-olefin) block copolymer, amorphous polypropylene, combinations of the above-mentioned polymers and polyethylene (e.g., LLDPE, LDPE, or HDPE), combinations of the above-mentioned polymers and polypropylene, and combinations thereof. Those olefin-based elastomers may be used alone or in combination thereof.

The density of the olefin-based elastomer is preferably 0.830 g/cm³ or more, more preferably 0.835 g/cm³ or more, still more preferably 0.840 g/cm³ or more, most preferably 0.845 g/cm³ or more. In addition, the density of the olefin-based elastomer is preferably 0.90 g/cm³ or less, more preferably 0.89 g/cm³ or less, still more preferably 0.88 g/cm³ or less. In one embodiment, the density of the olefin-based elastomer is preferably from 0.830 g/cm³ to 0.90 g/cm³, more preferably from 0.835 g/cm³ to 0.89 g/cm³, still more preferably from 0.840 g/cm³ to 0.89 g/cm³, particularly preferably from 0.845 g/cm³ to 0.88 g/cm³. When the density falls within the above-mentioned ranges, a post-treatment material for an absorbent article that can fix and hold the state in which the absorbent article is rolled up at the time of disposal can be provided. Further, the heat stability and storage stability of the post-treatment material can be improved. In addition, a process in the manufacture of the elastomer layer can be further simplified, and hence processing cost therefor can be further suppressed.

The melt flow rate (MFR) of the olefin-based elastomer at 230°C and 2.16 kgf is preferably 1.0 g/10 min or more, more preferably 2.0 g/10 min or more. In addition, the MFR of the olefin-based elastomer at 230°C and 2.16 kgf is preferably 25.0 g/10 min or less, more preferably 23.0 g/10 min or less, still more preferably 21.0 g/10 min or less, particularly preferably 20.0 g/10 min or less, most preferably 19.0 g/10 min or less. In one embodiment, the MFR of the olefin-based elastomer at 230°C and 2.16 kgf is preferably from 1.0 g/10 min to 25.0 g/10 min, more preferably from 2.0 g/10 min to 23.0 g/10 min, still more preferably from 2.0 g/10 min to 21.0 g/10 min, particularly preferably from 2.0 g/10 min to 20.0 g/10 min, most preferably from 2.0 g/10 min to 19.0 g/10 min. When the MFR falls within the above-mentioned ranges, a post-treatment material for an absorbent article that can fix and hold the state in which the absorbent article is rolled up at the time of disposal can be provided. Further, the heat stability and storage stability of the post-treatment material can be improved. In addition, a process in the manufacture of the elastomer layer can be further simplified, and hence processing cost therefor can be further suppressed.

An α-olefin-based elastomer is preferably used as the olefin-based elastomer. Of the α-olefin-based elastomers, at least one kind selected from an ethylene-based elastomer, a propylene-based elastomer, and a 1-butene-based elastomer is more preferably used. When such α-olefin-based elastomer is adopted as the olefin-based elastomer, a post-treatment material for an absorbent article that can fix and hold the state in which the absorbent article is rolled up at the time of disposal can be provided. Further, the heat stability and storage stability of the post-treatment material can be improved. In addition, a process in the manufacture of the elastomer layer and the resin layer can be further simplified, and hence processing cost therefor can be further suppressed.

A commercially available product may be used as the α-olefin-based elastomer. Examples of the commercially available product include "Tafmer" (trademark) series (e.g., Tafmer PN-2070 and Tafmer PN-3560) manufactured by Mitsui Chemicals, Inc., and "Vistamaxx" (trademark) series (e.g., Vistamaxx 3000, Vistamaxx 6202, and Vistamaxx 7010) manufactured by Exxon Mobil Corporation.

The α-olefin-based elastomer is preferably produced by using a metallocene catalyst. When the α-olefin-based elastomer produced by using a metallocene catalyst is adopted, a post-treatment material that can fix and hold the state in which the absorbent article is rolled up at the time of disposal can be provided. Further, the heat stability and storage stability of the post-treatment material can be improved. In addition, a process in the manufacture of the elastomer layer can be further simplified, and hence processing cost therefor can be further suppressed.

Examples of the styrene-based elastomer include a SIS-based elastomer and a SBS-based elastomer. In addition, a SIS-based elastomer having a specific molecular structure may be used as the styrene-based elastomer. Specific examples of the SIS-based elastomer include SIS-based elastomers including styrene-isoprene-styrene block copolymer molecular structures having different terminal styrene block chain lengths (hereinafter sometimes referred to as "specific SIS-based elastomers"). When such SIS-based elastomer is used, there can be provided a post-treatment material that can fix and hold the state in which the absorbent article is rolled up at the time of disposal, and that is more excellent in touch. The specific SIS-based elastomers may be used alone or in combination thereof.

Examples of the specific SIS-based elastomer include products available under the product names "Quintac 3390 (SL-159)" (styrene content=48 wt%) and "Quintac 3620" (styrene content=14 wt%) from Zeon Corporation.

The MFR of the specific SIS-based elastomer at 200°C and 5 kgf is preferably 5.0 g/10 min or more, more preferably 6.0 g/10 min or more, still more preferably 8.0 g/10 min or more. In addition, the MFR of the specific SIS-based elastomer is preferably 25.0 g/10 min or less, more preferably 23.0 g/10 min or less, still more preferably 21.0 g/10 min or less, particularly preferably 20.0 g/10 min or less, most preferably 18.0 g/10 min or less. In one embodiment, the MFR of the specific SIS-based elastomer at 200°C and 5 kgf is preferably from 5.0 g/10 min to 25.0 g/10 min, more preferably from 6.0 g/10 min to 23.0 g/10 min, still more preferably from 8.0 g/10 min to 21.0 g/10 min, particularly preferably from 8.0 g/10 min to 20.0 g/10 min, most preferably from 8.0 g/10 min to 18.0 g/10 min. When the MFR falls within the above-mentioned ranges, there can be provided a post-treatment material that can fix and hold the state in which the absorbent article is rolled up at the time of disposal, and that is more excellent in touch. In addition, a manufacturing process for the elastomer layer can be further simplified, and hence processing cost therefor can be further suppressed.

The content of the elastomer resin serving as the main component in the elastomer layer is preferably 50 wt% or more, more preferably 70 wt% or more, still more preferably 90 wt% or more. In addition, the content of the elastomer resin serving as the main component is preferably 100 wt% or less, more preferably 95 wt% or less. When the content of the elastomer resin serving as the main component falls within the above-mentioned ranges, the elastomer layer can express a sufficient elastomeric characteristic.

The elastomer layer may contain any appropriate other component to the extent that the effects of the present invention are not impaired. Examples of such other component include any other polymer, a tackifier, a plasticizer, an antidegradant, a pigment, a dye, an antioxidant, an antistatic agent, a lubricant, a foaming agent, a heat stabilizer, a light stabilizer, an inorganic filler, and an organic filler. The number of kinds of those components may be only one, or two or more. The content of the other component in the elastomer layer is preferably 10 wt% or less, more preferably 7 wt% or less, still more preferably 5 wt% or less, particularly preferably 2 wt% or less, most preferably 1 wt% or less.

Any appropriate polymer may be used as the other polymer. There is used, for example, a non-elastomeric resin to be used for the surface layers to be described later. The non-elastomeric resin may be used in any appropriate amount. For example, the content of the non-elastomeric resin in the elastomer resin is 50 wt% or less, and the non-elastomeric resin is used so that the total content of the non-elastomeric resin and the elastomer resin becomes 100 wt%.

The thickness of the elastomer layer is preferably 20 µm or more, more preferably 30 µm or more. In addition, the thickness of the elastomer layer is preferably 200 µm or less, more preferably 160 µm or less, still more preferably 140 µm or less, particularly preferably 120 µm or less, most preferably 100 µm or less. In one embodiment, the thickness of the elastomer layer is preferably from 20 µm to 200 um, more preferably from 20 µm to 160 µm, still more preferably from 30 µm to 140 µm, particularly preferably from 30 µm to 120 µm, most preferably from 30 µm to 100 µm. When the thickness of the elastomer layer falls within such ranges, a post-treatment material that can fix and hold the state in which the absorbent article is rolled up at the time of disposal can be provided.

The number of the elastomer layers may be one, or two or more. When the elastomer layer is a laminate of two or more layers, the laminate may include a plurality of layers including the same elastomer, or a plurality of layers including different elastomers.

In one embodiment, the resin layer includes three layers. In this embodiment, the resin layer includes an intermediate layer and surface layers arranged on both the sides of the intermediate layer. The intermediate layer preferably contains a white pigment. The incorporation of the white pigment provides appearance performance suitable for a sanitary product.

When the resin layer includes three layers, at least one of the surface layers and the intermediate layer only needs to be a layer including the above-mentioned elastomer. The surface layers on both the sides of the intermediate layer may each include an elastomer resin, the surface layers on both the sides may each include a non-elastomeric resin, or a surface layer including an elastomer resin and a surface layer including a non-elastomeric resin may be arranged on one side of the intermediate layer and on the other side thereof, respectively. When the surface layers each include an elastomer resin, expansibility of the post-treatment material is improved, and hence the state in which a sanitary product is rolled up at the time of disposal is easily fixed and held. As a result, the sanitary product is more easily disposed of. When the surface layers each include a non-elastomeric resin, blocking can be suppressed when the resin layer is formed into a wound body. In addition, processability (slittability) of the resin layer can be improved. It is preferred that the intermediate layer include an elastomer resin, and the surface layers on both the sides of the intermediate layer each include a non-elastomeric resin.

Any appropriate resin may be used as the non-elastomeric resin. Herein, the non-elastomeric resin refers to a resin except the above-mentioned elastomer resin. A non-elastomeric olefin-based resin is preferably used as the non-elastomeric resin.

Examples of the non-elastomeric olefin-based resin include an α-olefin homopolymer, a copolymer of two or more kinds of α-olefins, block polypropylene, random polypropylene, and a copolymer of one or two or more kinds of α-olefins and any other vinyl monomer. A copolymerization form in any such copolymer is, for example, a block form or a random form.

Examples of the α-olefin include α-olefins each having 2 to 12 carbon atoms. Examples of such α-olefin include ethylene, propylene, 1-butene, and 4-methyl-1-pentene.

Examples of the α-olefin homopolymer include polyethylene (PE), homopolypropylene (PP), poly(1-butene), and poly(4-methyl-1-pentene). Examples of the polyethylene (PE) include low-density polyethylene (LDPE), linear low-density polyethylene (LLDPE), medium-density polyethylene (MDPE), and high-density polyethylene (HDPE). The structure of the homopolypropylene (PP) may be any one of isotactic, atactic, and syndiotactic structures.

Examples of the copolymer of two or more kinds of α-olefins include an ethylene/propylene copolymer, an ethylene/1-butene copolymer, an ethylene/propylene/1-butene copolymer, a copolymer of ethylene/α-olefin having 5 to 12 carbon atoms, and a copolymer of propylene/α-olefin having 5 to 12 carbon atoms.

Examples of the copolymer of one or two or more kinds of α-olefins and any other vinyl monomer include an ethylene/vinyl acetate copolymer, an ethylene/acrylic acid alkyl ester copolymer, an ethylene/methacrylic acid alkyl ester copolymer, and an ethylene-non-conjugated diene copolymer.

HDPE, LDPE, LLDPE, homopolypropylene (h-PP), block polypropylene (b-PP), random polypropylene (r-PP), and a mixture thereof are each preferably used as the non-elastomeric olefin-based resin. When any such resin is used, blocking can be suppressed when the resin layer is formed into a wound body. In addition, processability (slittability) of the resin layer can be improved. Further, a chemical odor from the post-treatment material can be suppressed. In addition, the post-treatment material can be satisfactorily bonded to the exterior body of the absorbent article.

In one embodiment, a propylene resin may be preferably used as the non-elastomeric resin, and random polypropylene is more preferably used. The content of the random polypropylene in the non-elastomeric resin is preferably 50 wt% or more, more preferably 70 wt% or more, still more preferably 90 wt% or more.

The resin layer may contain any appropriate other component to the extent that the effects of the present invention are not impaired. Examples of such other component include a releasing agent, a UV absorber, a heat stabilizer, a filler, a lubricant, a colorant (e.g., a dye), an antioxidant, a die drool inhibitor, an antiblocking agent, a foaming agent, and polyethyleneimine. The number of kinds of those components may one, or two or more. The content of the other component in the resin layer is preferably 10 wt% or less, more preferably 7 wt% or less, still more preferably 5 wt% or less, particularly preferably 2 wt% or less, most preferably 1 wt% or less.

When the elastomer layer includes the intermediate layer and the surface layers, the thickness of each of the intermediate layer and the surface layers may be set to any appropriate value so that the thickness of the elastomer layer may fall within the above-mentioned ranges. The thickness of the intermediate layer is preferably 18 µm or more, more preferably 28 µm or more. In addition, the thickness of the intermediate layer is preferably 94 µm or less, more preferably 56 µm or less, still more preferably 46 µm or less. In one embodiment, the thickness of the intermediate layer is preferably from 18 µm to 94 µm, more preferably from 28 µm to 56 µm. The thickness of each of the surface layers is preferably 1 µm or more. In addition, the thickness of each of the surface layers is preferably 4 µm or less, more preferably 3 µm or less, still more preferably 2 µm or less. In one embodiment, the thickness of each of the surface layers is preferably from 1 µm to 4 um, more preferably from 1 µm to 3 µm, still more preferably from 1 µm to 2 µm. The thicknesses of the surface layers arranged on both the sides of the intermediate layer may be preferably set to the same thickness.

### D. Bonding Portion

The bonding portion is a portion that is weldable by ultrasonic welding or thermal welding. When the bonding portion that is weldable by ultrasonic welding or thermal welding is adopted, the post-treatment material and the absorbent article can be strongly bonded to each other even when a flexible non-woven fabric is used as the exterior body of the absorbent article. In addition, when no pressure-sensitive adhesive or adhesive is used, the manufacturing process can be reduced, and hence an environmental load caused by a chemical substance can be reduced. For example, when a post-treatment material obtained by laminating a non-woven fabric layer including a non-woven fabric containing chemical fibers and a resin layer including an elastomer layer is used, a post-treatment material including the bonding portion can be provided.

The bonding portions are typically formed on both the end portions of the post-treatment material having a band shape in its lengthwise direction. The bonding portion may be set to any appropriate size (area). The area of the bonding portion is preferably 25 mm² or more, more preferably 30 mm² or more. In addition, the area of the bonding portion is preferably 200 mm² or less, more preferably 100 mm² or less. In one embodiment, the area of the bonding portion is preferably from 25 mm² to 200 mm², more preferably from 30 mm² to 100 mm². When the size of the bonding portion falls within the above-mentioned ranges, the post-treatment material and the absorbent article can be strongly bonded to each other, and hence the detachment of the post-treatment material from the absorbent article at the time of the post-treatment can be suppressed. When the bonding is performed by an embossed pattern to be described later, the size of a portion on which the embossed pattern is formed only needs to fall within the above-mentioned ranges.

The entire surface of the bonding portion may be bonded, or a bonding portion having the embossed pattern subjected to embossing treatment may be adopted. The embossed pattern may be set to any appropriate shape. Specific examples of the embossed pattern include a continuous lattice shape, a discontinuous lattice shape, a continuous curve shape, a discontinuous curve shape, a continuous zigzag shape, a discontinuous zigzag shape, a continuous linear shape, a discontinuous linear shape, a circular shape (e.g., a dot shape), an elliptical shape, a hollow circular shape, a hollow elliptical shape, an arc shape, and a hollow arc shape.

The ratio of the area of a portion welded by the embossed pattern to the area of the entirety of the bonding portion (hereinafter sometimes referred to as "embossing welding area ratio") is preferably 10% or more, more preferably 15% or more, still more preferably 20% or more, particularly preferably 30% or more. In addition, the embossing welding area ratio is preferably 100% or less, more preferably 80% or less, still more preferably 60% or less, particularly preferably 50% or less. In one embodiment, the embossing welding area ratio is preferably from 10% to 100%, more preferably from 15% to 80%, still more preferably from 20% to 60%, particularly preferably from 30% to 50%. When the embossing welding area ratio falls within the above-mentioned ranges, the post-treatment material and the absorbent article can be strongly bonded to each other, and hence the detachment of the post-treatment material from the absorbent article at the time of the post-treatment can be suppressed.

The depth of the embossing and the thickness of the portion welded by the embossed pattern may each be set to any appropriate value. The depth of the embossing is, for example, from 0.1 mm to 2.0 mm. In addition, the thickness of the portion welded by the embossed pattern is, for example, 100 µm or less.

### D-1. Ultrasonic Welding

The bonding with an ultrasonic wave may be performed by any appropriate method. When the bonding portion is formed by the ultrasonic bonding, the post-treatment material and the absorbent article can be more strongly bonded to each other. Further, the occurrence of a unique odor derived from an adhesive and a pressure-sensitive adhesive is further suppressed, and an environmental load by the adhesive and the pressure-sensitive adhesive can be suppressed. In addition, the manufacturing process can be reduced.

The ultrasonic welding is specifically performed by arranging members to be bonded (e.g., the post-treatment material and the exterior body of the absorbent article) between a part for feeding vibration energy through use of an ultrasonic wave (part generally referred to as "horn") and a roll-shaped part (generally referred to as "anvil"). The horn is typically arranged vertically above the members to be bonded and the anvil. The horn typically vibrates at from 20,000 Hz to 40,000 Hz to transfer energy typically in the form of frictional heat to the members to be bonded under pressure. Part of at least one of the members to be bonded is softened or melted by the frictional heat and the pressure, and hence the layers are bonded to each other.

A pressing force between the horn and the anvil in ultrasonic welding is preferably from 100 N to 1,500 N, more preferably from 300 N to 1,300 N, still more preferably from 500 N to 1,100 N, particularly preferably from 700 N to 1,000 N. When the pressing force between the horn and the anvil in the ultrasonic welding falls within the above-mentioned ranges, the flexibility of the post-treatment material is further improved, and a more satisfactory texture thereof can be achieved. In addition, the production rate of the post-treatment material can be further improved.

In one embodiment, the ultrasonic welding is performed by a method generally known as "continuous ultrasonic fusion bonding." The continuous ultrasonic fusion bonding is typically used for sealing members to be bonded that can be supplied into a bonding apparatus in a substantially continuous manner. In the continuous ultrasonic fusion bonding, the horn is typically fixed and the members to be bonded move directly below the horn. In one kind of continuous ultrasonic fusion bonding, the fixed horn and a rotating anvil surface are used. During the continuous ultrasonic fusion bonding, the members to be bonded are pulled between the horn and the rotating anvil. The horn typically extends in its lengthwise direction toward the members to be bonded, and its vibration moves along the horn in its axial direction to the materials.

In another embodiment, the horn is a rotation type, has a cylindrical shape, and rotates about its lengthwise direction axis. Input vibration is present in the axial direction of the horn and output vibration is present in the radial direction of the horn. The horn is arranged so as to be close to the anvil, and the anvil can also typically rotate so that the members to be bonded may pass a space between cylindrical surfaces at a line velocity substantially equal to the tangential velocity of the cylindrical surfaces.

The ultrasonic fusion bonding is described in, for example, JP 2008-526552 A, JP 2010-195044 A, JP 2013-231249 A, JP 2015-16294 A, and US 5976316 A, and the contents of the disclosures are incorporated herein by reference.

In one embodiment, the above-mentioned rotating anvil surface preferably has an embossed pattern. Specific examples of such embossed pattern are as described above. The embossed pattern of the rotating anvil surface is disclosed in, for example, JP 2017-65253 A. The contents of the disclosure are incorporated herein by reference.

The shape of the tip portion (portion to be brought into contact with the members to be bonded) of a convex portion for forming the embossed pattern may be set to any appropriate shape, and is, for example, a circular shape. When an embossed pattern having a circular tip portion is used, the diameter of the circular portion is preferably 0.4 mm or more, more preferably 0.45 mm or more, still more preferably 0.5 mm or more. In addition, the diameter of the circular portion is preferably 1 mm or less, more preferably 0.9 mm or less, still more preferably 0.8 mm or less. When the diameter falls within the above-mentioned ranges, both of the productivity and adhesive strength of the post-treatment material can be achieved. The embossed depth of the pattern is preferably 0.5 mm or more. In addition, the embossed depth is preferably 1.5 mm or less, more preferably 1 mm or less. When the embossed depth falls within the above-mentioned ranges, both of the productivity and adhesive strength of the post-treatment material can be achieved.

In addition, from the viewpoint of stabilizing the quality of the post-treatment material to be obtained, the ultrasonic welding is preferably performed while the temperature of the anvil is controlled so as to be any appropriate temperature. When variation in temperature of the anvil is excessively large, there is a problem in that variation in adhesive strength also occurs to make it impossible to obtain a post-treatment material having stable quality. The temperature of the anvil may be set to, for example, from 5°C to 90°C in terms of absolute temperature, and temperature control is performed so that the temperature of the anvil may be the preset temperature±5°C. When the temperature of the anvil falls within the above-mentioned ranges, a reduction in productivity due to condensation or the like is prevented, and adverse effects on product characteristics such as the curing of the non-woven fabric and the resin layer including an elastomer layer can be prevented.

### D-2. Bonding by Thermal Welding

The bonding by thermal welding may be performed by any appropriate method. For example, a thermally welded portion may be formed by forming the bonding portion with a heated embossed pattern roll. A heating temperature is preferably from 150°C to 220°C, more preferably from 160°C to 210°C, still more preferably from 170°C to 200°C.

### E. Method of manufacturing Post-treatment Material

The post-treatment material may be manufactured by any appropriate method. Examples thereof include: (1) a method including laminating the resin layer including an elastomer layer formed by extrusion from the T-die of an extruder and the non-woven fabric layer separately drawn from a roll body; (2) a method including simultaneously extruding and laminating the resin layer including an elastomer layer and the non-woven fabric layer; (3) a method including bonding the resin layer including an elastomer layer and the non-woven fabric layer, which have been prepared separately from each other, to each other with an adhesive; and (4) the bonding of the resin layer including an elastomer layer and the non-woven fabric layer to each other by heat lamination or an ultrasonic wave. A preferred example of a method for the bonding is bonding with an ultrasonic wave (ultrasonic fusion bonding). When the ultrasonic bonding is adopted, no pressure-sensitive adhesive is used, and hence a step of forming a pressure-sensitive adhesive layer can be omitted. Accordingly, the emission amount of carbon dioxide can be reduced. In addition, the methods for the heat lamination and the ultrasonic bonding that can reduce an environmental load caused by a chemical substance and can reduce the contamination on another material at the manufacturing stage are as described above.

In one embodiment of the present invention, the non-woven fabric layer and the resin layer having an elastomer layer are bonded to each other via any appropriate pressure-sensitive adhesive. A hot-melt pressure-sensitive adhesive is preferably used. The use of the hot-melt pressure-sensitive adhesive reduces the need for the addition of a tackifier as a component for the resin layer. Thus, for example, the extrusion stability of the layers is improved, and hence a problem in that the tackifier adheres to a forming roll can be suppressed. In addition, a problem in that a manufacturing line is contaminated by, for example, volatile matter derived from the tackifier can be suppressed.

The hot-melt pressure-sensitive adhesive may be applied to the entire surface of the non-woven fabric layer, or may be applied to part of the non-woven fabric layer. When the hot-melt pressure-sensitive adhesive is applied to a part on the non-woven fabric layer, the hot-melt pressure-sensitive adhesive is preferably applied so as to include at least the end portions of the non-woven fabric layer. The hot-melt pressure-sensitive adhesive may be applied to the non-woven fabric layer and/or the resin layer by any appropriate method.

Any appropriate pressure-sensitive adhesive may be used as the hot-melt pressure-sensitive adhesive. Examples thereof include a hot-melt pressure-sensitive adhesive containing a styrene-based elastomer, and a hot-melt pressure-sensitive adhesive containing an olefin-based polymer. Of those, a hot-melt pressure-sensitive adhesive containing a styrene-based polymer is preferred. Examples of such styrene-based polymer include a polymer including a SIS structure, a polymer including a SBS structure, hydrogenated products thereof, and blends thereof. The hot-melt pressure-sensitive adhesives may be used alone or in combination thereof. In addition, when the hot-melt pressure-sensitive adhesive contains the styrene-based polymer, the number of kinds of the styrene-based polymers may be only one, or two or more. When the hot-melt pressure-sensitive adhesive contains the styrene-based polymer, the content of the styrene-based polymer in the hot-melt pressure-sensitive adhesive is preferably from 10 wt% to 90 wt%.

The hot-melt pressure-sensitive adhesive may further contain any appropriate other component. Examples of such other component include liquid paraffin, a tackifier, an antioxidant, a UV absorber, a light stabilizer, and a fluorescent agent. The number of kinds of such other components may be only one, or two or more. Those other components are each used in any appropriate amount.

The post-treatment material may be further subjected to activation treatment after the lamination. The activation treatment may be performed by any appropriate method. Specific examples thereof include stretching treatment in the widthwise direction and/or lengthwise direction of the post-treatment material, and treatment in which a fiber structure of part of the region of the non-woven fabric layer is mechanically broken. The post-treatment material subjected to the activation treatment is further improved in elongation, and hence the post-treatment of the rolled-up absorbent article can be more easily performed.

### F. Absorbent Article

An absorbent article according to an embodiment of the present invention includes an exterior body and the above-mentioned post-treatment material. When the absorbent article includes the above-mentioned post-treatment material, an absorbent article on which the post-treatment can be easily performed can be provided. Examples of the absorbent article include a pants-type diaper, a tape-type diaper, and a women's sanitary product. Of those, a pants-type diaper is preferred. The absorbent article that is the pants-type diaper is specifically described below.

The structure of the pants-type diaper is not particularly limited, and any of well-known structures may be utilized. An example of the pants-type diaper is a diaper including: an absorbing pad; and an exterior body that supports the absorbing pad on an inner surface thereof and has a pants-type external shape. The absorbing pad includes at least: a liquid-permeable top sheet; a liquid-impermeable back sheet; and an absorber arranged therebetween, which absorbs and holds a body fluid that has permeated through the top sheet. The exterior body is divided into, for example, a ventral external member to be brought into abutment with mainly a ventral portion of a wearer, a dorsal external member to be brought into abutment with mainly a dorsal portion of the wearer, and a crotch member interposed between the ventral external member and the dorsal external member, the crotch member being to be brought into abutment with mainly a crotch portion of the wearer, the crotch member supporting mainly the absorbing pad. The dorsal external member may include a plurality of portions. As a portion for forming the dorsal external member, there are given, for example, a back-sheet external member and a waist portion external member. The post-treatment material may be bonded to the back-sheet external member, or may be bonded to the waist portion external member. In addition, one end portion of the post-treatment material may be bonded to the back-sheet external member and the other end portion may be bonded to the waist portion external member. An example of the exterior body is an exterior body including a side seal portion obtained by bonding both edges of the ventral external member and the dorsal external member in their widthwise direction, the exterior body including a waist opening portion and a pair of leg opening portions in the widthwise direction. In this case, the ventral external member and the dorsal external member may be substantially symmetric with respect to the crotch member.

The outermost surface of the exterior body is preferably a non-woven fabric, more preferably a non-woven fabric having a density of from 15 kg/m³ to 120 kg/m³. When such non-woven fabric is the outermost surface of the exterior body, an absorbent article having satisfactory texture and being excellent in flexibility can be provided. The density of the non-woven fabric is preferably 20 kg/m³ or more, more preferably 23 kg/m³ or more. In addition, the density of the non-woven fabric is preferably 115 kg/m³ or less, more preferably 110 kg/m³ or less. When the density of the non-woven fabric falls within the above-mentioned ranges, the absorbent article is excellent in appearance and texture. In one embodiment, the density of the non-woven fabric is preferably from 20 kg/m³ to 115 kg/m³, more preferably from 23 kg/m³ to 110 kg/m³.

The non-woven fabric serving as the outermost surface of the exterior body is preferably a spunlace non-woven fabric, a carded non-woven fabric, an air-through non-woven fabric, or a spunjet non-woven fabric. Examples of the non-woven fabric to be used for the exterior body include the same ones as those of the non-woven fabric to be used for the post-treatment material described above. The non-woven fabric to be used for the post-treatment material and the non-woven fabric to be used for the exterior body may be identical to or different from each other.

In the absorbent article according to the embodiment of the present invention, the post-treatment material is bonded thereto by ultrasonic welding or thermal welding. A method for the ultrasonic welding or the thermal welding is as described above.

### Examples

The present invention is specifically described below by way of Examples. However, the present invention is by no means limited to these Examples. Evaluation methods in Examples and the like are as described below. In addition, the term "part(s)" means "part(s) by weight" and the term "%" means "wt%" unless otherwise stated.

### [Example 1]

A resin layer (hereinafter sometimes referred to as "elastic film") was extrusion-molded with an extrusion T-die molding machine including three layers in two types (A layer/B layer/A layer). Specifically, 100 parts by weight of an olefin-based resin 1 (manufactured by SCG Performance Chemicals Co., Ltd., product name: P756C) was loaded into each of the A layers of the extruder, and 46.5 parts by weight of an olefin-based elastomer 1 (manufactured by Exxon Mobil Corporation, product name: Vistamaxx 6202), 46.5 parts by weight of an olefin-based elastomer 2 (manufactured by Mitsui Chemicals, Inc., product name: Tafmer PN-3560), and 7 parts by weight of a white pigment (titanium oxide, manufactured by Ampacet Corporation, product name: White PE MB 111413) were loaded into the B layer of the extruder, followed by the extrusion molding of an elastic film 1 having a basis weight of 45 g/m² (A layer/B layer/A layer=4%/92%/4%). The extrusion was performed under the following temperature conditions: A layer: 200°C, B layer: 230°C, and die temperature: 230°C.

A non-woven fabric A (polypropylene (PP) carded type, basis weight=24 gsm) was directly laminated on each of both the surfaces of the resultant elastic film 1, and ultrasonic bonding was performed so that the film and the fabrics were completely bonded to each other. The ultrasonic bonding was performed by subjecting a non-woven fabric 1, the elastic film, and a non-woven fabric 2 in a three-layer laminated state to ultrasonic fusion lamination with an ultrasonic fusion facility (manufactured by Herrmann Ultraschall, apparatus name: MICROBOND (ULTRABOND 48:20)) at a frequency of 20 kHz (output intensity: 1,800 W) and a line velocity of 100 m/min. An embossed pattern roll used in the fusion was a roll having a dot pattern having a fusion area of 8% and a diameter of 0.7 mm. After that, activation treatment was performed to provide a post-treatment material 1.

### [Example 2]

A post-treatment material 2 was obtained in the same manner as in Example 1 except that an elastic film was produced by using 100 parts by weight of an olefin-based resin 1 (manufactured by SCG Performance Chemicals Co., Ltd., product name: P756C) for each of the A layers of the extruder, and 46.5 parts by weight of a styrene-based elastomer 1 (manufactured by Zeon Corporation, product name: Quintac 3620), 46.5 parts by weight of a styrene-based elastomer 2 (manufactured by Zeon Corporation, product name: Quintac 3390), and 7 parts by weight of a white pigment (titanium oxide, manufactured by Ampacet Corporation, product name: White PE MB 111413) for the B layer of the extruder.

### [Example 3]

A post-treatment material 3 was obtained in the same manner as in Example 2 except that the elastic film and the non-woven fabrics were thermally welded. The thermal welding was performed by subjecting the non-woven fabric 1, the elastic film, and the non-woven fabric 2 in a three-layer laminated state to thermal welding lamination at a treatment temperature of 200°C, a linear pressure of 160 N/mm, and a treatment speed of 50 m/min. An embossed pattern roll used in the fusion was a roll having a dot pattern having a fusion area of 8% and a diameter of 0.7 mm. Next, activation treatment was performed to provide the post-treatment material 3.

### [Example 4]

A post-treatment material 4 was obtained in the same manner as in Example 2 except that the elastic film and the non-woven fabrics were laminated via an adhesive. The lamination with the adhesive was performed by bonding the non-woven fabrics to both the surfaces of the elastic film with a roll. In this case, a hot-melt adhesive (manufactured by Nitto Bento Bant 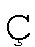 ilik, product name: AC280) was applied (15 g/m²) to a side to which the non-woven fabric was bonded, in a stripe manner (width of the adhesive: 1 mm, interval: 1 mm). Next, activation treatment was performed to provide the post-treatment material 4.

### [Example 5]

A post-treatment material 5 was obtained in the same manner as in Example 2 except that a non-woven fabric B (PP spunlace type, basis weight=35 gsm) was laminated on one surface of the elastic film instead of the non-woven fabric A.

### [Example 6]

A post-treatment material 6 was obtained in the same manner as in Example 2 except that the non-woven fabric B was laminated on each of both the surfaces of the elastic film.

### [Example 7]

A post-treatment material 7 was obtained in the same manner as in Example 2 except that a non-woven fabric C (PP-polyethylene terephthalate (PET) spunlace type (content of PP fiber: 50 wt%), basis weight=35 gsm) was laminated on each of both the surfaces of the elastic film.

### [Example 8]

A post-treatment material 8 was obtained in the same manner as in Example 2 except that a non-woven fabric D (PP spunlace type, basis weight=25 gsm) was laminated on each of both the surfaces of the elastic film.

### (Comparative Example 1)

A post-treatment material C1 was obtained in the same manner as in Example 2 except that the non-woven fabric was not laminated (the resin layer was used alone).

### (Comparative Example 2)

A post-treatment material C2 was obtained in the same manner as in Example 2 except that a non-woven fabric E (PP spunbonded type, basis weight=20 gsm) was laminated on each of both the surfaces of the elastic film.

### (Comparative Example 3)

A post-treatment material C3 was obtained in the same manner as in Example 4 except that a non-woven fabric F (cotton spunlace type, basis weight=35 gsm) was laminated on each of both the surfaces of the elastic film.

### (Comparative Example 4)

An attempt was made to produce a post-treatment material in the same manner as in Example 2 except that the non-woven fabric F was laminated on each of both the surfaces of the elastic film. However, the non-woven fabrics and the elastic film were not bonded to each other, and hence the post-treatment material was not obtained.

### (Comparative Example 5)

A post-treatment material C5 was obtained in the same manner as in Example 2 except that a non-woven fabric G (PE-PET air-through type, basis weight=25 gsm) was used as the non-woven fabric layer.

### (Reference Example)

An attempt was made to laminate the post-treatment material produced in Example 1 on a diaper by applying an adhesive (manufactured by Nitto Bento Bant 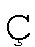 ilik, product name: AC280) to the post-treatment material so that its thickness after drying became 40 g/m². The post-treatment material after the lamination was peeled from the diaper, and hence its use as the post-treatment material was difficult.

### [Evaluation]

The post-treatment materials obtained in Examples, Comparative Examples, and Reference Example were subjected to the following evaluations. The results are shown in Table 1.

### <Tensile Test>

The resultant post-treatment materials were evaluated for their tensile characteristics by being subjected to the following test. The post-treatment materials were each cut into a piece having a width of 10 mm and a length of 10 cm so that its direction (CD) perpendicular to a film machine direction (MD) served as a long side. Thus, samples were obtained. Each of the samples was set in a tensile testing machine (manufactured by ZwickRoell GmbH & Co KG, product name: Z0005 1 kN) at a chuck-to-chuck distance of 50 mm, followed by pulling at a tensile speed of 500 mm/min until its breakage. A maximum strength, a breaking elongation, and an elongation at 2 N at this time were measured. The evaluations were performed on five samples each, and average values were used.

### <10-minute Holding Test>

The resultant post-treatment materials were evaluated for their 10-minute holding properties by being subjected to the following test. The post-treatment materials were each cut into a piece having a width of 10 mm and a length of 10 cm so that its direction (CD) perpendicular to a film machine direction (MD) served as a long side. Thus, samples were obtained. Each of the samples was set in a tensile testing machine (manufactured by ZwickRoell GmbH & Co KG, product name: Z0005 1 kN) at a chuck-to-chuck distance of 50 mm. The sample was pulled at a tensile speed of 500 mm/min until an elongation of 100% was achieved. After that, the sample was held for 10 minutes at an elongation of 100%, and then the distance was returned to the initial chuck-to-chuck distance. A stress after 10 minutes (stress after extension) at this time was measured. In addition, deformation after 10 minutes was calculated by measuring a deformation amount of the sample after the holding for 10 minutes, with a ruler. The evaluations were performed on five samples each, and average values were used.

### <Adhesive Property to Diaper Exterior Body>

The post-treatment material cut out into a width of 10 mm in the MD direction was bonded to a commercially available carded non-woven fabric (24 g/m², PP) with an ultrasonic welding machine (manufactured by Honda Electronics Co., Ltd., product name: SONAC-37, welding metal part YK01). A case in which the peeling of the bonded post-treatment material from the non-woven fabric was not found at the time of the pulling of the material in the CD direction was evaluated as being good (satisfactory), and a case in which the bonding was not able to be performed and a case in which the peeling was easily found were evaluated as being poor (room for improvement).

### <Appearance>

In the evaluation for the adhesive property to a diaper exterior body, a case in which the outermost surface (side visible from the outside) of the laminate was formed of a non-woven fabric and was in a fluffy and soft state at the time of bonding to the non-woven fabric was evaluated as being very good (very satisfactory), a case in which the outermost surface was formed of a non-woven fabric but felt hard was evaluated as being good (satisfactory), and a case in which the outermost surface was formed of a film was evaluated as being poor (room for improvement) .

### <Ease of Disposal>

A sample having a maximum strength of 3 N/10 mm or more, a breaking elongation of 150% or more, and an elongation at 2 N of 65% or more was evaluated as being excellent (best), a sample having a maximum strength of 3 N/10 mm or more, a breaking elongation of 150% or more, and an elongation at 2 N of 60% or more was evaluated as being very good (very satisfactory), a sample having a maximum strength of 3 N/10 mm or more, a breaking elongation of 150% or more, and an elongation at 2 N of 50% or more was evaluated as being good (satisfactory), and a sample having a maximum strength, a breaking elongation, and an elongation at 2 N below the above-mentioned ranges was evaluated as being poor (room for improvement).

### <Holding Characteristic>

A sample having a stress after 10 minutes of 1 N/10 mm or more and a deformation after 10 minutes of 50% or less was evaluated as being good (satisfactory), and a sample except the foregoing was evaluated as being poor (room for improvement).

### <Thickness of Non-woven Fabric>

A cross-section of the post-treatment material was photographed at a magnification of 100 times with a digital microscope "VHX-1000" manufactured by Keyence Corporation, and the thickness of the non-woven fabric was measured with the image analysis software of the digital microscope. In the measurement of the thickness, when the resin layer (elastic film) and the non-woven fabric was brought into contact with each other, the boundary between the resin layer and the non-woven fabric was defined as the outermost surface, or when the non-woven fabrics were brought into contact with each other, in the upper portion and the lower portion of the cross-section of the non-woven fabric, parallel lines drawn so as to have 10 points of intersection with fibers were defined as an upper side and a lower side, respectively, and a length between the upper side and the lower side was adopted as the thickness of the non-woven fabric. The thickness of the non-woven fabric was measured at a number of samples of 3 or more, and an average of the measured values was adopted as the thickness of the non-woven fabric.

**Table 1**

| | | Examp le 1 | Examp le 2 | Examp le 3 | Examp le 4 | Examp le 5 | Examp le 6 | Examp le 7 | Examp le 8 | Compara tive Example 1 | Compara tive Example 2 | Compara tive Example 3 | Compara tive Example 4 | Compara tive Example 5 | Reference Example |
|---|---|---|---|---|---|---|---|---|---|---|---|---|---|---|---|
| Non-woven fabric 1 | Manuf actur ing metho d | Carde d | Carde d | Carde d | Carde d | Carde d | Spunl ace | Spunl ace | Spunl ace | - | Spunbon ded | Spunlac e | Spunlac e | Air-through | Carded |
| | Compo sitio n | PP | PP | PP | PP | PP | PP | PP-PET | PP | - | PP | Cotton | Cotton | PE-PET | PP |
| | Basis weigh t [g/m² ] | 24 | 24 | 24 | 24 | 24 | 35 | 35 | 25 | - | 20 | 35 | 35 | 25 | 24 |
| | Thick ness [mm] | 0.40 | 0.49 | 0.37 | 0.33 | 0.36 | 0.38 | 0.50 | 0.28 | - | 0.16 | 0.53 | 0.59 | 2.12 | 0.42 |
| | Densi ty [kg/m ³] | 60 | 49 | 66 | 72 | 67 | 92 | 70 | 89 | | 125 | 66 | 59 | 12 | 57 |
| Film | Kind | Olefi n | Styre ne | Styre ne | Styre ne | Styre ne | Styre ne | Styre ne | Styre ne | Styrene | Styrene | Styrene | Styrene | Styrene | Olefin |
| | Basis weigh t [g/m² ] | 45 | 45 | 45 | 45 | 45 | 45 | 45 | 45 | 45 | 45 | 45 | 45 | 45 | 45 |
| Non-woven fabric 2 | Manuf actur ing metho d | Carde d | Carde d | Carde d | Carde d | Spunl ace | Spunl ace | Spunl ace | Spunl ace | - | Spunbon ded | Spunlac e | Spunlac e | Air-through | Carded |
| | Compo sitioN | PP | PP | PP | PP | PP | PP | PP-PET | PP | - | PP | Cotton | Cotton | PE-PET | PP |
| | Basis weigh t [g/m² ] | 24 | 24 | 24 | 24 | 35 | 35 | 35 | 25 | - | 20 | 35 | 35 | 25 | 24 |
| | Thick ness [mm] | 0.44 | 0.47 | 0.38 | 0.32 | 0.40 | 0.41 | 0.52 | 0.30 | - | 0.14 | 0.55 | 0.62 | 2.25 | 0.44 |
| | Densi ty [kg/m ³] | 55 | 51 | 63 | 75 | 88 | 85 | 67 | 83 | - | 143 | 64 | 56 | 11 | 55 |
| Lamination method | | Ultra sonic | Ultra sonic | Heat | Adhes ive | Ultra sonic | Ultra sonic | Ultra sonic | Ultra sonic | - | Ultraso nic | Adhesiv e | Ultraso nic | Ultraso nic | Ultrasoni c |
| Basis weight of laminate [g/m²] | | 93 | 93 | 93 | 113 | 104 | 115 | 115 | 95 | Styrene | 85 | 135 | Poor Not bonded | 95 | 93 |
| Method for bonding to diaper | | Ultra sonic | Ultra sonic | Ultra sonic | Ultra sonic | Ultra sonic | Ultra sonic | Ultra sonic | Ultra sonic | Ultraso nic | Ultraso nic | Ultraso nic | - Unevalu able | Ultraso nic | Adhesive |
| Adhesive property to diaper | | Good | Good | Good | Good | Good | Good | Good | Good | Poor Peeled | Good | Poor Not bonded | - Unevalu able | Good | Poor Peeled |
| Appearance | | Very good | Very good | Very good | Very good | Very good | Very good | Very good | Very good | Poor | Good | - Unevalu able | - Unevalu able | Good | - Unevaluab le |
| Maximum strength [N/10 mm] | | 6.1 | 5.7 | 5.5 | 6.7 | 6.3 | 9.1 | 6.0 | 4.8 | Poor Unevalu able | 6.3 | - Unevalu able | - Unevalu able | 2.3 | - Unevaluab le |
| Breaking elongation [%] | | 202.7 | 209.4 | 201.1 | 401.9 | 276.7 | 557.8 | 387.3 | 243.4 | Poor Unevalu able | 102.3 | - Unevalu able | - Unevalu able | 232.6 | - Unevaluab le |
| Elongation at 2 N [%] | | 58.2 | 54.7 | 53.3 | 50.8 | 64.1 | 70.1 | 85.9 | 99.2 | Poor Unevalu able | 23.4 | - Unevalu able | - Unevalu able | 120.3 | - Unevaluab le |
| Ease of disposal | | Good | Good | Good | Good | Very good | Excel lent | Excel lent | Excel lent | Poor Unevalu able | Poor | - Unevalu able | - Unevalu able | Poor | - Unevaluab le |
| Stress after extension [N/10 mm] | | 1.3 | 2.0 | 2.0 | 2.0 | 1.4 | 1.3 | 1.2 | 1.1 | Poor Unevalu able | 2.5 | - Unevalu able | - Unevalu able | 1.0 | - Unevaluab le |
| Deformation after 10 minutes [%] | | 30.2 | 24.0 | 24.4 | 29.0 | 17.0 | 20.0 | 18.0 | 18.0 | Unevalu able | | - Unevalu able | - Unevalu able | 20.0 | Unevaluab le |
| Holding characteristic | | Good | Good | Good | Good | Good | Good | Good | Good | Poor Unevalu able | Good | - Unevalu able | - Unevalu able | Good | - Unevaluab le |

### Industrial Applicability

The post-treatment material for an absorbent article of the embodiment of the present invention can be used in any appropriate article in which the effects of the present invention can be effectively utilized. A typical example of the absorbent article of the present invention is a diaper (a pants-type diaper or a tape-type diaper).

### Reference Signs List

- **100**: post-treatment material for absorbent article

- **10**: resin layer including elastomer layer
- **20a**: non-woven fabric layer
- **20b**: non-woven fabric layer
- **30**: bonding portion
- **200**: absorbent article

## Claims

1. A post-treatment material for an absorbent article, the post-treatment material comprising:
a non-woven fabric layer including a non-woven fabric having a density of from 15 kg/m³ to 120 kg/m³ and containing chemical fibers;
a resin layer including an elastomer layer; and
a bonding portion to be bonded to an exterior body of an absorbent article,
wherein the non-woven fabric layer serves as an outermost surface of the post-treatment material for an absorbent article, and
wherein the bonding portion is a portion configured to be weldable by ultrasonic welding or thermal welding.

2. The post-treatment material for an absorbent article according to claim 1, wherein the non-woven fabric is a spunlace non-woven fabric, a carded non-woven fabric, an air-through non-woven fabric, or a spunjet non-woven fabric.

3. The post-treatment material for an absorbent article according to claim 1 or 2, wherein the chemical fibers are olefin-based resin fibers.

4. The post-treatment material for an absorbent article according to claim 3, wherein the chemical fibers are polypropylene-based resin fibers.

5. The post-treatment material for an absorbent article according to claim 4, wherein the non-woven fabric contains 30 wt% or more of the polypropylene-based resin fibers.

6. The post-treatment material for an absorbent article according to any one of claims 1 to 5, wherein the elastomer layer contains a styrene-based elastomer or an olefin-based elastomer.

7. The post-treatment material for an absorbent article according to claim 6, wherein the elastomer layer contains the olefin-based elastomer.

8. The post-treatment material for an absorbent article according to claim 7, wherein the olefin-based elastomer is a propylene-based elastomer.

9. The post-treatment material for an absorbent article according to any one of claims 1 to 8, wherein the post-treatment material has foldable structures.

10. The post-treatment material for an absorbent article according to any one of claims 1 to 9, wherein the post-treatment material has a band shape and a length of from 50 mm to 250 mm.

11. The post-treatment material for an absorbent article according to any one of claims 1 to 10, wherein the post-treatment material has a basis weight of from 50 g/m² to 150 g/m².

12. The post-treatment material for an absorbent article according to any one of claims 1 to 11, wherein the post-treatment material has a breaking elongation of from 150% to 600%.

13. The post-treatment material for an absorbent article according to any one of claims 1 to 12, wherein the post-treatment material has a maximum strength of 2 N/10 mm or more.

14. The post-treatment material for an absorbent article according to any one of claims 1 to 13, wherein the post-treatment material has a stress after extension of 0.5 N/10 mm or more.

15. The post-treatment material for an absorbent article according to any one of claims 1 to 14, wherein the post-treatment material has an elongation at a load of 2 N of 30% or more.

16. An absorbent article, comprising:
an exterior body; and
the post-treatment material for an absorbent article of any one of claims 1 to 15.

17. The absorbent article according to claim 16,
wherein the exterior body includes a non-woven fabric in an outermost surface thereof, and
wherein the non-woven fabric has a density of from 15 kg/m³ to 120 kg/m³.

18. The absorbent article according to claim 16 or 17, wherein the absorbent article is a pants-type diaper.

19. A method of manufacturing an absorbent article, the method comprising:
laminating a non-woven fabric layer including a non-woven fabric having a density of from 15 kg/m³ to 120 kg/m³ and containing chemical fibers, and a resin layer including an elastomer layer to produce a laminate; and
bonding the laminate and an exterior body of an absorbent article to each other by ultrasonic welding or thermal welding.
